# EUROPÄISCHE PATENTSCHRIFT

(11) **EP 1 767 158 B1**
(45) Veröffentlichungstag und Bekanntmachung des Hinweises auf die Patenterteilung: **26.03.2008**
(21) Anmeldenummer: 06020098.7
(22) Anmeldetag: 26.09.2006
(51) Int. Cl.: A61B 17/11, C12N 5/00, A61F 2/08

(54) **Verbindungselement für Gewebe**
Tissue connector
Connecteur pour tissu biologique

(30) Priorität: 27.09.2005 DE 102005045988
(43) Veröffentlichungstag der Anmeldung: 28.03.2007
(73) Patentinhaber: Seide, Klaus, 21465 Reinbek (DE); Müller, Jörg, 21244 Buchholz (DE)
(72) Erfinder: Seide, Klaus, 21465 Reinbek (DE); Müller, Jörg, 21244 Buchholz (DE)

(56) Entgegenhaltungen:
- WO-A-96/31160
- US-A- 3 786 817
- US-A- 4 306 561

## Beschreibung

Nach der Durchtrennung von Nervengewebe, insbesondere des Rückenmarks (Querschnittslähmung) bilden die verletzten Nervenstrukturen zunächst an den Enden Aussprossungen als Versuch einer Heilung. Gleichzeitig werden Substanzen freigesetzt, welches dies unterdrücken und es findet eine Narbenbildung statt. Bekannt ist, dass sowohl die heilungshindernden Substanzen als auch die Narbenbildung durch Medien so beeinflusst werden können, dass insbesondere die Heilung des Rückenmarks möglich werden könnte. Während die biochemischen Vorgänge schon therapeutisch genutzt werden könnten, erfolgt die mechanische Adaptation in der Regel mit Nähten, welche eine Ausrichtung nur größerer Bündel ermöglichen. Es fehlt ein Verfahren zur optimalen mechanischen Adaptation des Gewebes im Submillimeterbereich. Darüber hinaus ist von entscheidender Bedeutung, dass die o.g. biologisch aktiven Substanzen an diese Gewebsverbindung heran, bzw. in diese hinein gebracht werden können. WO 96831160 offenbart ein röhrenförmiger Verbindungselement für zwei Gewebeenden.

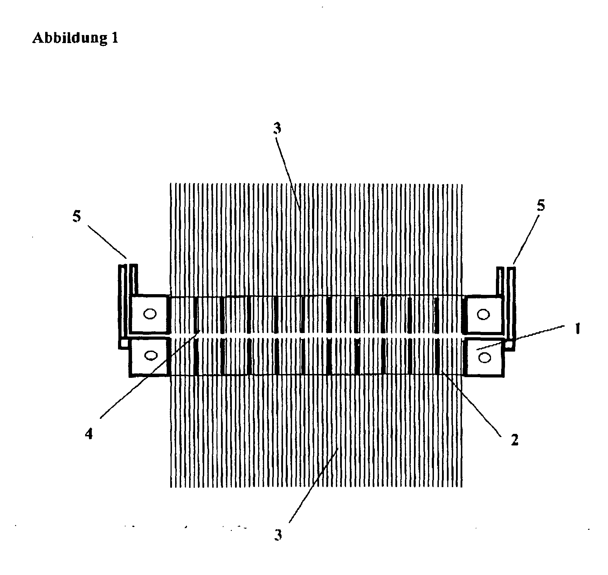

Gegenstand der Erfindung ist eine Anordnung, die dieses Problem lösen soll. Dazu wird - hergestellt vorzugsweise mit Verfahren der Mikro- und Nanotechnologie - gemäß Abbildung 1 ein Verbindungselement eingesetzt, das auf den jeweiligen durchtrennten Gewebeseiten (3) Submillimeter große, röhrenförmige Strukturen mit hohem Aspektverhältnis (Kapillaren) bei geringen verbleibenden Wandstärken aufweist (2) und das im Zentrum eine Kanalstruktur (4) enthält, die über Zufuhr- und Abfuhrkanäle (5) das Gewebe mit biologisch aktiven Substanzen versorgen kann. Darüber hinaus dienen diese Kanalstrukturen und Anschlüsse dazu, das Gewebe beiderseits in die kapillarähnlichen Strukturen einzusaugen, um den zu überbrückenden Abstand zwischen den Gewebeenden so klein wie erforderlich zu gestalten. Um die Haftung in diesen Kapillaren zu erhöhen und das gerichtete Wachstum zusätzlich zu fördern, können ihre Innenwände zusätzlich mit Beschichtungen versehen werden, die Wachstum und Haftung fördern. Darüber hinaus ist es Gegenstand der Erfindung, dass dort zusätzliche Anordnungen (1) integriert sind, die eine mechanische Verankerung ermöglichen. Dimensionen und Form von Kapillaren und Verbindungselement sollten auf die Geometrie und Struktur der zu verbindenden Gewebeteile eingestellt sein. Größere Gewebebereiche sind dazu ggf. in parallel verlaufenden kleineren Segmenten in den Kapillaren zu führen. Dazu ist deren Wandstärke zu minimieren, was vorzugsweise mit hexagonalen, ggf. auch quadratischen, dreieckigen oder runden Querschnitten erreicht wird.

Eine weitere Möglichkeit, die dieses Element bietet, ist die Integration von elektrischen Kontakten an der Verbindungsstelle, um dort elektrisch zu stimulieren oder z.B. Nervensignale abzuleiten.

Als Materialien für diese Verbindungselemente eignen sich für die Kapillaranordnung neben dem in der Mikrostrukturtechnik verwendeten Silizium ggf. in Kombination mit Glas für die Kanalstrukturen insbesondere in z.B. mit LIGA- oder SIGA-Verfahren erzeugten Formeinsätzen abgeformte Polymere vorzugsweise aus bioverträglichen, möglicherweise elastischen oder vom Körper resorbierbaren Polymeren. Diese können vorzugsweise aus zwei symmetrischen Halbstrukturen bestehen, die jeweils die Kapillarstruktur 4 und Versorgungssowie die Zu- bzw. Abfuhrkanäle 5 enthalten.. Die Auskleidung zur Erhöhung der Bioverträglichkeit, Haftung und des Wachstums wie auch zu einer zeitlichen Stabilisierung resorbierbarer Materialien eignen sich z.B. vorzugsweise im Plasma oder durch Tauchverfahren abgeschiedene Schichten wie Polytetrafluoräthylen, Silikon oder Polyäthylen. Falls für eine mechanische Stabilisierung erforderlich kann das Verbindungselement mit einer in der Längsrichtung trennbaren Manschette eingepasst werden.

Über die beschriebene Anwendung hinaus eignet sich ein solches Verbindungselement auch für andere natürliche Gewebe, wie z. Sehnen, Bänder und Muskeln sowie für zur Verbindung technischer Fasern.

## Patentansprüche

1. Verbindungselement für Gewebe, **dadurch gekennzeichnet, dass** es aus zwei zueinander ausgerichteten Segmenten (1) mit parallel angeordneten kapillarähnlichen, röhrenförmigen Strukturen (2) besteht, in welche von zwei Seiten Fasern eines Gewebes (3) eingeführt werden können, und es dazwischen ein Hohlraumsystem (4) aufweist, welches mit den Kapillarenstrukturen und der Umgebung kommuniziert (5).

2. Verbindungselement für Gewebe nach Anspruch 1, **dadurch gekennzeichnet, dass** im Hohlraumsystem ein Unterdruck erzeugt werden kann, um die Gewebe in die Kapillarstrukturen einzubringen.

3. Verbindungselement für Gewebe nach Ansprüchen 1 und 2, **dadurch gekennzeichnet, dass** durch dass Hohlraumsystem Flüssigkeiten geleitet werden können.

4. Verbindungselement für Gewebe nach Anspruch 3, **dadurch gekennzeichnet, dass** es sich um biologisch wirksame Substanzen (z.B. Medikamente o.ä.) handelt.

5. Verbindungselement für Gewebe nach Ansprüchen 1 bis 4, **dadurch gekennzeichnet, dass** die Kapillaren eine möglichst geringe Wandstärke aufweisen und vorzugsweise hexagonal, quadratisch, dreieckig, ggfs auch rund sind.

6. Verbindungselement für Gewebe nach Ansprüchen 1 bis 5, **dadurch gekennzeichnet, dass** die Kapillaren und der Verbinder einer der Verbindungsstelle in Struktur und Dimension angepasste Form haben.

7. Verbindungselement für Gewebe nach Ansprüchen 1 bis 6, **dadurch gekennzeichnet, dass** die Innenstruktur der Kapillaren ein Zurückgleiten verhindert vorzugsweise durch Rauhigkeit, Widerhaken oder andere Formgebung

8. Verbindungselement für Gewebe nach Ansprüchen 1 bis 7, **dadurch gekennzeichnet, dass** die Kapillaren mit bioverträglichen, wachstumsfördernden Materialien ausgekleidet sind.

9. Verbindungselement für Gewebe nach Ansprüchen 1 bis 8 **dadurch gekennzeichnet, dass** das System getrennt und nach Einfügen der Gewebe, ggf. unter Verwendung eines weiteren Trägers verbunden werden kann.

10. Verbindungselement für Gewebe nach Ansprüchen 1 bis 9, **dadurch gekennzeichnet, dass** es mit elektrischen Anschlüsse versehen ist, um Signale abzuleiten oder zu stimulieren.

11. Verbindungselement für Gewebe nach Ansprüchen 1 bis 10 **dadurch gekennzeichnet, dass** auf das Hohlraumsystem verzichtet und nur die Verbindungsfunktion genutzt wird.

12. Verbindungselement für Gewebe nach Ansprüchen 1 bis 11 **dadurch gekennzeichnet, dass** es zur mechanischen Stabilisierung der Verbindung mit einer längs trennbaren steigen Manschette umgeben wird.

13. Verbindungselement für Gewebe nach Ansprüchen 1 bis 12 **dadurch gekennzeichnet, dass** es aus gut strukturierbaren Materialien der Mikrosystemtechnik wie Silizium und Glas besteht

14. Verbindungselement für Gewebe nach Ansprüchen 1 bis 12 **dadurch gekennzeichnet, dass** es aus vorzugsweise bioverträglichen Polymer-Materialien besteht.

15. Verbindungselement für Gewebe nach Ansprüchen 1 bis 12 **dadurch gekennzeichnet, dass** es aus resorbierbaren Materialien besteht.

16. Verbindungselement für Gewebe nach Ansprüchen 1 bis 12 und 15 **dadurch gekennzeichnet, dass** zur zeitlichen Stabilisierung der resorbierbaren Materialien eine Oberflächenbeschichtung erfolgt.

17. Verbindungselement für Gewebe nach Anspruch 1 bis 16 **dadurch gekennzeichnet, dass** mit Methoden der Mikrosystemtechnik in direkter Strukturierung hergestellt wird.

18. Verbindungselement für Gewebe nach Anspruch 1 bis 16 **dadurch gekennzeichnet, dass** mit Methoden der Mikrosystemtechnik in Abformtechnik hergestellt wird.

19. Verbindungselement für Gewebe nach Ansprüchen 1 bis 18, **dadurch gekennzeichnet, dass** es für die Verbindung von Rückenmarksgewebe eingesetzt wird.

20. Verbindungselement für Gewebe nach Ansprüchen 1 bis 18, **dadurch gekennzeichnet, dass** es für die Verbindung von Nerven eingesetzt wird.

21. Verbindungselement für Gewebe nach Ansprüchen 1 bis 18, **dadurch gekennzeichnet, dass** es für die Verbindung von anderen natürlichen Geweben (z.B. Sehnen, Bänder, Muskeln, Haut, innere Organe) eingesetzt wird.

22. Verbindungselement für Gewebe nach Ansprüchen 1 bis 18, **dadurch gekennzeichnet, dass** es für die Verbindung von nicht natürlichen Fasern eingesetzt wird.

## Claims

1. Tissue linking element, **characterized by** consisting of two segments (1) aligned to one another, each consisting of parallel aligned capillary-like, tubular structures (2), in which tissue fibers (3) can be inserted from two sides, and a compartment (4) in-between that communicates with the capillary structures and surroundings (5).

2. Tissue linking element as in Claim 1 **characterized by** creation of an underpressure within the compartment in order to insert tissue into the capillary system.

3. Tissue linking element as in Claims 1 and 2 **characterized by** the ability of the compartment to channel fluids.

4. Tissue linking element as in Claim 3 **characterized by** the fluids to be biologically active substances (i.e., pharmaceuticals, etc.).

5. Tissue linking element as in Claims 1 through 4 **characterized by** capillaries exhibiting least possible wall thickness that are preferably hexagonal, quadratic, triangular, or if necessary round.

6. Tissue linking element as in Claims 1 through 5 **characterized by** capillaries and connectors that are adapted in structure and dimension to the periphery.

7. Tissue linking element as in Claims 1 through 5 **characterized by** the interior capillary structure that prevents backsliding preferably through coarseness, barbs, or other shaping.

8. Tissue linking element as in Claims 1 through 7 **characterized by** capillaries being lined with biologically compatible, growth-promoting materials.

9. Tissue linking element as in Claims 1 through 8 **characterized by** the system being able to be separated and, after insertion of tissue, connected using an additional component.

10. Tissue linking element as in Claims 1 through 9 **characterized by** electrical connections, so that signals can be registered or stimulated.

11. Tissue linking element as in Claims 1 through 10 **characterized by** dispensing of the compartment and only using the connecting function.

12. Tissue linking element as in Claims 1 through 11 **characterized by** mechanical stabilization of connections covered by a lengthwise separable stiff sleeve.

13. Tissue linking element as in Claims 1 through 12 **characterized by** consisting of well structurable microsystem technology materials such as silicon and glass.

14. Tissue linking element as in Claims 1 through 12 **characterized by** preferably consisting of bio-compatible polymer materials.

15. Tissue linking element as in Claims 1 through 12 **characterized by** consisting of bioabsorbable materials.

16. Tissue linking element as in Claims 1 through 12 **characterized by** a surface coating for long term stabilization of bioabsorbable materials

17. Tissue linking element as in Claims 1 through 16 **characterized by** the use of microsystem technology methods in direct structuring.

18. Tissue linking element as in Claims 1 through 16 **characterized by** use of microsystem technology replication methods.

19. Tissue linking element as in Claims 1 through 18 **characterized by** usage for connecting spinal cord tissue.

20. Tissue linking element as in Claims 1 through 18 **characterized by** usage for connecting nerves.

21. Tissue linking element as in Claims 1 through 18 **characterized by** being used for connecting other natural tissues (e.g., tendons, ligaments, muscles, skin, organs).

22. Tissue linking element as in Claims 1 through 18 **characterized by** being used for connecting non biologic fibers.

## Revendications

1. élément de liaison tissulaire, **caractérisée par le fait qu'**il se compose de deux segments (1) alignés ensemble avec des structures (2) en parallèle, identiques à celles capillaires et cylindriques, dans lesquelles les fibres tissulaire (3) peuvent être introduites des deux côtés et qu'il présente entre les deux une cavité (4), communiquant(5) avec les structures capillaires et l'environnement.

2. élément de liaison tissulaire selon la revendication 1, **caractérisée par le fait qu'**un système sous pression peut être créé dans la cavité, pour introduire les tissus dans les structures capillaires.

3. élément de liaison tissulaire selon les revendications 1 et 2, **caractérisé par le fait que** les liquides peuvent être introduits par la cavité.

4. élément de liaison tissulaire selon la revendication 3, **caractérisé par le fait qu'**il s'agit de substances biologiquement actives (par ex. médicaments ou id.).

5. élément de liaison tissulaire selon les revendications 1 à 4, **caractérisé par le fait que** les capillaires ont une épaisseur de paroi aussi fine que possible et sont de préférence hexagonales, carrées, triangulaires ou rondes.

6. élément de liaison tissulaire selon les spécifications 1 à 5, **caractérisé par le fait que** les capillaires et l'élément jonctionnel ont une forme adaptée en structure et dimension.

7. élément de liaison tissulaire selon les spécifications 1 à 6, **caractérisé par le fait que** l'intérieur de la structure des capillaires empêche un glissement en arrière par sarugosité, crochet ou tout autre forme.

8. élément de liaison tissulaire selon les revendications 1 à 7, **caractérisé par le fait que** les capillaires sont revêtus de matériaux biocompatibles, aptes à favoriser la croissance.

9. élément de liaison tissulaire selon les revendications 1 à 8, **caractérisé par le fait que** le système est séparé et après insertion des tissus, le cas échéant, peut être lié en utilisant un autre support.

10. élément de liaison tissulaire selon les revendications 1 à 9, **caractérisé par le fait qu'**il est muni de raccordements électriques pour transmettre ou stimuler des signaux.

11. élément de liaison tissulaire selon les revendications 1 à 10 **caractérisé par le fait qu'**on renonce à la cavité et que seulement la fonction de liaison est utilisée.

12. elément de liaison pour les tissus selon les revendications 1 à 11 **Caractérisé par le fait qu'**il est entouré pour stabilisation mécanique de la liaison avec une manchette verticale séparable en longueur.

13. élément de liaison tissulaire selon les revendications 1 à 12 **caractérisé par** sa composition de matériaux bien structurables des micro-systèmes, tels que le silicium et le verre.

14. élément de liaison tissulaire selon les revendications 1 à 12 **caractérisé** sa composition de matériaux polymères biocompatibles.

15. élément de liaison tissulaire selon les revendications 1 à 12 **caractérisé par** sa composition de matériaux résorbables.

16. élément de liaison tissulaire selon les revendications 1 à 12 et 15 **caractérisé par** un revêtement de surface utilisé pour stabiliser dans le temps les matériaux résorbables.

17. élément de liaison pour les tissus selon les revendications 1 à 16, **caractérisé par** l'utilisation de méthode en structuration directe des micro-systèmes pour son autofabrication.

18. élément de liaison tissulaire selon les revendications 1 à 16, **caractérisé** nécessitant des techniques de modelage des micro-systemes.

19. élément de liaison tissulaire selon les revendications 1 à 18, **caractérisé par** son utilisation pour la jonction tissulaire de la moelle épinière.

20. élément de liaison tissulaire selon les revendications 1 à 18, **caractérisé par** son utilisation pour la connexion nerveuse.

21. élément de liaison tissulaire selon les revendications 1 à 18, **caractérisé par le fait qu'**il est utilisé pour la liaison d'autres tissus naturels (par exemple les tendons, les ligaments, les muscles, la peau, les viscères).

22. élément de liaison tissulaire selon les revendications 1 à 18, **caractérisé par le fait qu'**il est utilisé pour la liaison de fibres non naturelles.
